# EUROPEAN PATENT APPLICATION

(11) **EP 1 582 871 A1**
(43) Date of publication of application: **05.10.2005**
(21) Application number: 04700767.9
(22) Date of filing: 08.01.2004
(51) Int. Cl.: G01N 33/561, G01N 27/447

(54) **COMPOSITION CONTAINING PARTICLE SURFACE CHARGE CONTROL AGENT, PARTICLE SEPARATING METHOD USING SAME, PARTICLE SEPARATOR**

(30) Priority: 10.01.2003 JP 2003003941
(71) Applicant: MATSUSHITA ELECTRIC INDUSTRIAL CO., LTD., Kadoma-shi, Osaka 571-8501 (JP)
(72) Inventor: NAKAYAMA, Hiroshi, Matsushita Electric Ind.Co.,Ltd, Kadoma-shi, Osaka 571-8501 (JP)
(74) Representative: Gassner, Wolfgang
(86) International application number: PCT/JP2004/000067
(87) International publication number: WO 2004/063752

(57) **Abstract**

The present invention is a composition for modifying an amount of charges on a surface of a target particle in a sample and separating or quantitatively determining the target particle in the sample, based on the modified surface charge amount, and relates to the composition comprising a charge control agent having a positive or negative charge in a solution and being capable of specifically binding to the target particle. The present invention also relates to a method of separating or quantitatively determining the target particle in the sample. The above method comprises the steps of mixing a sample containing the target particle and a charge control agent specifically binding to the target particle and having a positive or negative charge in the sample, and binding the charge control agent to the target particle; and separating or quantitatively determining the target particle provided with the charge control agent bound thereto, based on a surface charge modified by the binding of the charge control agent, by applying a voltage or current to the sample resulting from the mixing.

## Description

### TECHNICAL FIELD

The present invention relates to a composition for controlling a surface charge of a particle, a particle separating method using the same, and a particle separator.

### BACKGROUND ART

As a method of separating particles such as cells, bacteria, viruses, and organic or inorganic polymers, methods using an ion-exchange liquid chromatography and an electrophoresis, respectively, are well known. In these methods, each particle, which is an object being tested, is separated by the electric interaction with the solid phase or the electrical mobility by utilizing a difference in a surface charge of each particle. For example, there is a report describing a separation of T lymph cells and B lymph cells by utilizing a capillary electrophoresis device (Japanese Journal of Medical Electronics and Biological Engineering, Vol. 15, No. 10 (2001), pp. 12-16). However, the above two cells are not completely separated from each other. Thus, a simple and accurate particle separation technology is demanded.

On the other hand, there is a well-known method of modifying a surface of a particle and artificially changing the surface charge thereof.

For example, a method (SDS electrophoresis) of separating proteins by attaching sodium dodecyl sulfate (abbreviated as SDS) to the surface of the proteins by hydrophobic linkages for analyzing the proteins by the electrophoresis is well known.

Also, a method of injecting a labeled substrate (for example, labeled by fluorescence or radioactive substance) into a cell or homogenized cell lysates for measuring cell activity is known. When the labeled substance is injected into the cell, an enzyme existing in the cell converts (for example, phosphorylates) the substrate, whereby the surface charge of the substrate changes. As a result, the electrical mobility of the substrate is changed before and after the injection of the substrate into the cell or homogenized cell lysates, whereby it is possible to detect cell activity by measuring the amount of changed substrates using the capillary electrophoresis (for example, see US2002/0142323A1 and US2002/0037542A1).

### DISCLOSURE OF THE INVENTION

In the case where the conventional technology described in the above-mentioned Japanese Journal of Medical Electronics and Biological Engineering, Vol. 15, No. 10 (2001), pp.2-7 is used, a particle being tested is separated based only on the surface charge thereof, and this separation from impurities is often poor. This is because there is no significant difference among substances composing the surfaces of the different particles, and each substance has a relatively low charge such that there is no significant variation in the electrical mobility of the particles and the particles are poorly electrically separated from each other.

On the other hand, in the above-described SDS electrophoresis, SDS is nonspecifically bound to the surface of a protein. Thus, in principle, the amount of SDS to be bound varies with the size of the protein, whereby it is impossible to artificially control the binding amount. Also, SDS is an ionic surface-active agent, and denatures biological materials such as proteins. Thus, the above-described method is applied only to a denatured protein.

Also, in the above-described cell activity measuring method, the surface charge of a substrate is changed using an endoenzyme, and the amount of surface charges is controlled depending on the amount and type of the endoenzyme, whereby it is impossible to perform artificial charge control. Also, this method uses only an endoenzyme and a substrate reacting with the endoenzyme, whereby it is impossible to modify a cell surface and change a cell surface charge.

The present invention provides the solution to the problems of the above-stated conventional technologies.

Based on one aspect, the present invention provides a composition for modifying an amount of charges on a surface of a target particle in a sample and separating or quantitatively determining the target particle in the sample, based on the modified surface charge amount. The composition comprises a charge control agent having a positive or negative charge in a solution and being capable of specifically binding to the target particle.

In a preferable embodiment of the composition of the present invention, the charge control agent specifically binds to a biological functional substance selected from a group consisting of an organic polymer, a protein, sugar, lipid, and nucleic acid, which are present on the surface of the target particle.

In a preferable embodiment of the composition of the present invention, the charge control agent comprises a group selected from a group consisting of a carboxylic acid group, a phosphate group, asulfonicgroup, a phenol group, an alcohol group, a tertiary amino group, and a quaternary amino group.

In a preferable embodiment of the composition of the present invention, the charge control agent comprises a protein, a peptide, or nucleic acid, which is capable of specifically binding to the target particle. Preferably, the nucleic acid is an aptamer or a functional equivalent thereof. More preferably, the charge control agent further comprises a marker having a positive or negative charge in a solution.

In a further preferable embodiment of the composition of the present invention, the charge control agent is a complex composed of an antibody or a functional equivalent thereof, which is capable of specifically binding to the biological functional substance, and the marker bound thereto.

In another further preferable embodiment, the charge control agent is a complex composed of a ligand for a receptor present on the surface of the target particle or a functional equivalent thereof and the marker bound thereto. Preferably, the ligand is a peptide hormone, a growth factor, cytokine, or catecholamine.

In another further preferable embodiment, the charge control agent is a complex composed of an aptamer or a functional equivalent thereof and the marker bound thereto.

Preferably, the marker is a dyeing marker, a gold colloid, or latex.

Preferably, the dyeingmarker is aminoethyl4-azidebenzamide trisodium salt or N-(3-triethlyammoniumpropyl)-4-(4-(dioctadecylamino) styryl) pyridiniumdi-4-chlorobenzenesulfonate.

In a preferable embodiment of the composition of the present invention, the charge control agent is reversibly bound to the target particle.

In a preferable embodiment of the composition of the present invention, the charge control agent is specifically bound to the target particle by an ionic bond or a hydrogen bond.

In a preferable embodiment of the composition of the present invention, the target particle is a cell selected from a group consisting of a white blood cell, a lymphocyte, a platelet, and a red blood cell. Preferably, the lymphocyte is a T cell, a B cell, or an NK cell.

The composition of the present invention is preferably used for testing an immune function of a subject.

The composition of the present invention is preferably used for measuring a level of fatigue or stress of a subject.

The composition of the present invention is preferably used for determining whether or not a subject is infected with a virus.

In a preferable embodiment of the composition of the present invention, the target particle is a bacterium, a virus, or a fungus. Preferably, the bacterium is selected from a group consisting of Escherichia coliform bacillus, salmonella, Yersinia enterocolitica, Vibrio parahaemolyticus, bacillus cereus, Campylobacter, Clostridium perfringens, and Staphylococcus aureus.

The composition of the present invention is preferably used for prevention and investigation of food poisoning.

In another aspect, the present invention provides a manufacturing method of a charge control agent used for modifying an amount of charges on a surface of a target particle in a sample and separating or quantitatively determining the target particle in the sample, based on the modified surface charge amount.

The manufacturing method of the charge control agent of the present invention is characterized in that it comprises a step of binding a marker having a positive or negative charge in a solution to a protein or nucleic acid, or a functional equivalent thereof, which is capable of specifically binding to the target particle.

In a preferable embodiment of the manufacturing method of the charge control agent of the present invention, the protein or the nucleic acid, or the functional equivalent thereof is specifically bound to a biological functional substance selected from a group consisting of an organic polymer, a protein, sugar, lipid, and nucleic acid, which are present on the surface of the target particle.

In a further preferable embodiment of the manufacturing method of the charge control agent of the present invention, the protein is an antibody.

In a further preferable embodiment of the manufacturing method of the charge control agent of the present invention, the nucleic acid is an aptamer.

In a further preferable embodiment of the manufacturing method of the charge control agent of the present invention, the protein is a ligand for a receptor present on the surface of the target particle.

In a preferable embodiment of the manufacturing method of the charge control agent of the present invention, the marker comprises a group selected from a group consisting of a carboxylic acid group, a phosphate group, a sulfonic group, a phenol group, an alcohol group, a tertiary amino group, and a quaternary amino group.

In a preferable embodiment of the manufacturing method of the charge control agent of the present invention, the marker is a dyeing marker, a gold colloid, or latex. Preferably, the dyeing marker is aminoethyl4-azidebenzamide trisodium salt or N-(3-triethlyammoniumpropyl)-4-(4-(dioctadecylamino) styryl) pyridiniumdi-4-chlorobenzenesulfonate.

In a preferable embodiment of the manufacturing method of the charge control agent of the present invention, the target particle is a cell selected from a group consisting of a white blood cell, a lymphocyte, a platelet, and a red blood cell.

In another preferable embodiment of the manufacturing method of the charge control agent of the present invention, the target particle is a bacterium, a virus, or a fungus.

In a preferable embodiment of the manufacturing method of the charge control agent of the present invention, a ratio or an amount of the marker to be bound to the protein or the nucleic acid, or the functional equivalent thereof is adjustable.

In still another aspect, the present invention provides a method of separating or quantitatively determining a target particle in a sample. The method is characterized in that it comprises the steps of mixing a sample containing the target particle and a charge control agent specifically binding to the target particle and having a positive or negative charge in the sample, and binding the charge control agent to the target particle; and separating or quantitatively determining the target particle provided with the charge control agent bound thereto, based on a surface charge modified by the binding of the charge control agent, by applying a voltage or current to the sample resulting from the mixing.

Preferably, the mixing step is separately performed for a plurality of types of particles.

Preferably, the mixing step is performed for mixing a plurality of types of particles with respective charge control agents which are different from each other.

In a preferable embodiment of the separation or quantitative determination method of the present invention, the target particle is a cell selected from a group consisting of a white blood cell, a lymphocyte, a platelet, and a red blood cell, or a microorganism selected from a group consisting of a bacterium, a virus, and a fungus.

In a further preferable embodiment of the separation or quantitative determination method of the present invention, the charge control agent is bound to a biological functional substance selected from a group consisting of an organic polymer, a protein, sugar, lipid, and nucleic acid, which are present on the surface of the target particle.

In a further preferable embodiment of the separation or quantitative determination method of the present invention, the charge control agent comprises a protein, a peptide, or nucleic acid, which is capable of specifically binding to the target particle.

In a further preferable embodiment of the separation or quantitative determination method of the present invention, the nucleic acid is an aptamer or a functional equivalent thereof.

In a further preferable embodiment of the separation or quantitative determination method of the present invention, the charge control agent further comprises a marker having a positive or negative charge in a solution.

In a further preferable embodiment of the separation or quantitative determination method of the present invention, the charge control agent is a complex composed of an antibody or a functional equivalent thereof, which is capable of specifically binding to the biological functional substance, and the marker bound thereto.

In still another preferable embodiment of the separation or quantitative determination method of the present invention, the charge control agent is a complex composed of a ligand for a receptor present on the surface of the target particle or a functional equivalent thereof and the marker bound thereto. More preferably, the ligand is a peptide hormone, a growth factor, cytokine, or catecholamine.

In still another preferable embodiment of the separation or quantitative determination method of the present invention, the charge control agent is a complex composed of an aptamer or a functional equivalent thereof and the marker bound thereto.

In a further preferable embodiment of the separation or quantitative determination method of the present invention, the marker is a dyeing marker, a gold colloid, or latex. More preferably, the dyeing marker is aminoethyl4-azidebenzamide trisodium salt or N-(3-triethlyammoniumpropyl)-4-(4-(dioctadecylamino) styryl) pyridiniumdi-4-chlorobenzenesulfonate.

In still another aspect, the present invention provides an instrument for separating or quantitatively determining a target particle in a sample. The instrument is characterized in that it comprises mixing means for mixing a sample containing the target particle and a charge control agent specifically binding to the target particle and having a positive or negative charge in the sample, and binding the charge control agent to the target particle; and separation/quantitative determination means for separating or quantitatively determining the target particle provided with the charge control agent bound thereto, based on a surface charge modified by the binding of the charge control agent, by applying a voltage or current to the sample resulting from the mixing.

In a preferable embodiment of the instrument of the present invention, a plurality of injectionmeans for separately injecting the sample containing the target particle and the charge control agent are further included.

In a preferable embodiment of the instrument of the present invention, the target particle is a cell selected from a group consisting of a white blood cell, a lymphocyte, a platelet, and aredbloodcell, or a microorganism selected from a group consisting of a bacterium, a virus, and a fungus.

In a preferable embodiment of the instrument of the present invention, the charge control agent is bound to a biological functional substance selected from a group consisting of an organic polymer, a protein, sugar, lipid, and nucleic acid, which are present on the surface of the target particle.

In a preferable embodiment of the instrument of the present invention, the charge control agent comprises a protein, a peptide, or nucleic acid, which is capable of specifically binding to the target particle. Preferably, the nucleic acid is an aptamer or a functional equivalent thereof.

In a preferable embodiment of the instrument of the present invention, the charge control agent further comprises a marker having a positive or negative charge in a solution.

In a preferable embodiment of the instrument of the present invention, the charge control agent is a complex composed of an antibody or a functional equivalent thereof, which is capable of specifically binding to the biological functional substance, and the marker bound thereto.

In a preferable embodiment of the instrument of the present invention, the charge control agent is a complex composed of a ligand for a receptor present on the surface of the target particle or a functional equivalent thereof and the marker bound thereto. Preferably, the ligand is a peptide hormone, a growth factor, cytokine, or catecholamine.

In a preferable embodiment of the instrument of the present invention, the charge control agent is a complex composed of an aptamer or a functional equivalent thereof and the marker bound thereto.

In a preferable embodiment of the instrument of the present invention, the marker is a dyeing marker, a gold colloid, or latex. Preferably, the dyeing marker is aminoethyl4-azidebenzamide trisodium salt or N-(3-triethlyammoniumpropyl)-4-(4-(dioctadecylamino) styryl) pyridiniumdi-4-chlorobenzenesulfonate.

In the present specification, a "functional equivalent" of a protein such as an antibody or a peptide such as a peptide hormone is a polypeptide or a peptide capable of binding to the same target molecule (e.g., an antigen or a cell surface receptor) as that of an original protein or peptide with similar specificity and affinity, although its amino acid sequence is different from that of the original protein or peptide due to substitution, addition, or deletion of one or more amino acids. Furthermore, assume that an original protein or peptide, or a functional equivalent thereof modified by a peptide modifying group such as phosphorylation (Ser, Thr, Tyr), acetylation (N terminal, Lys), or C terminal amidation is also included in the above "functional equivalent" as long as it is capable of binding to the same target molecule as that of the original protein or peptide with similar specificity and affinity.

In the present specification, a "functional equivalent" of an aptamer is nucleic acid (RNA or DNA) capable of binding to the same target molecule as that of an original aptamer with similar specificity and affinity, although its base sequence is different from that of the original aptamer due to substitution, addition, or deletion of one or more bases. Furthermore, assume that a modified original aptamer or functionally-equivalent nucleic acid (e.g., whose 5' terminal is modified by fluorochrome) is also included in the above "functional equivalent" as long as it is capable of binding to the same target molecule as that of the original aptamer with similar specificity and affinity.

In the present invention, "specific binding to a target particle" means binding to a target particle with higher affinity compared to other particles. In the case where Ka (association constant) = [A-B] / ([A] · [B]) (note that A and B are two types of molecules and A-B is a complex of A and B) is used, it is preferable that specific affinity be at least approximately 10⁵, 10⁶, 10⁷, 10⁸, 10⁹, or 10¹⁰M⁻¹.

The present invention provides a simple and accurate particle separation technology. The present invention enables effective control of a surface charge of a particle by using a surface charge control agent, and realizes an accurate and simple particle separation and measurement.

The present invention enables a charge to be supplied specifically to a target particle. Thus, even if sizes of the target particle and other molecules are substantially the same, it is possible to perform separation by electrophoresis.

The charge control agent of the present invention is reversibly bound to a target particle, and an ionic surface-active agent such as SDS is not used in the present invention. Thus, collection of the separated/detected cells and bacteria is realized without damage.

### BRIEF DESCRIPTION OF THE DRAWINGS

FIG. 1 is an illustration showing an outline of the principle of the present invention.
FIG. 2 is a chromatogram illustrating a separation of helper T cells and killer T cells by the present invention.
FIG. 3 is a chromatogram illustrating a separation of bacteria cells by the present invention.
FIG. 4 is an illustration showing an outline of a particle separator according to the present invention.

### BEST MODE FOR CARRYING OUT THE INVENTION

The present invention relates to a composition for separating or quantitatively determining target particles in a sample by modifying the amount of charges on the surface of the target particles and utilizing the modified amount of surface charges. The composition includes a charge control agent having a positive or negative charge in a solution and being capable of specifically binding to the target particles.

The charge control agent of the present invention preferably has an aqueous ionizable group such as a sulfonic group, a phosphate group, an amino group, an alcohol group, a phenol group, or a carboxylic acid group, and has a function being capable of binding to other substances. Appropriate charge control agents used in the present invention include, for example, a complex formed by an antibody specifically binding to an antigen present on the cell surface and a marker having a positive or negative charge in a solution, or an aptamer specifically binding to other substances and having a charge in a solution.

In general, the target particles being tested in the present invention include blood cells being tested, such as red blood cells, white blood cells, and platelets, and cells of microorganisms such as bacteria, viruses, and fungi, etc., but they are not limited thereto. The present invention can be applied to an arbitrary substance existing in a sample as a particle.

Hereinafter, an embodiment of the present invention will be described.

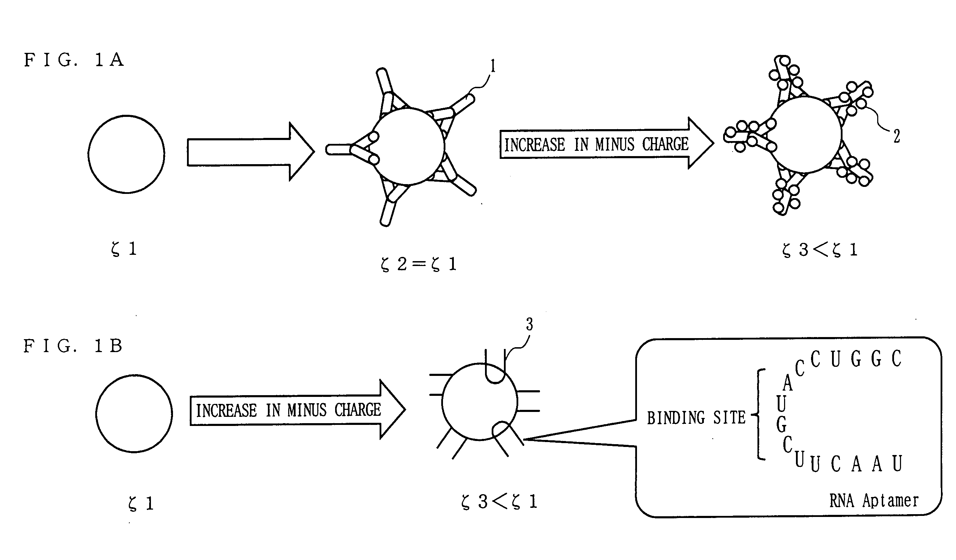

In FIG. 1, the principle of the present invention is outlined. In general, when there is relative motion between a solid (a particle) and a liquid, a difference between a potential of the outermost surface (boundary) of a layer (stationary layer) at which the liquid flows along the solid wall and a potential of the rest of the liquid is referred to as a ζ potential or an electrokinetic potential. In other words, the ζ potential controls electrokinetic phenomena occurring when there is relative motion between the solid and the liquid.

FIG. 1A schematically illustrates an exemplary case in which a labeled antibody is used as a charge control agent, and FIG. 1B schematically illustrates an exemplary case in which an aptamer is used as a charge control agent.

As illustrated in FIG. 1A, when a particle such as a blood cell being tested (indicated as a circle on the left side of each of A and B of FIG. 1. In general, blood cells and microorganic cell bodies are negatively charged) moves in a solution, the particle generates ζ1, which is a ζ potential unique to the particle. When an antibody molecule 1, which specifically binds to the particle by an immune response, is bound to the particle (illustrated in the middle of FIG. 1A), the resultant antibody-particle complex also generates a ζ potential ζ2 while moving in the solution. In general, the antibody molecule 1 does not have a significant effective potential. Thus, ζ2 is substantially equal to ζ1. Here, in the case where the antibody molecule 1 has a marker 2 having a charge (in the exemplary case as illustrated in FIG. 1A, a negative charge), a ζ potential ζ3, which is generated when the labeled antibody-particle complex moves in the solution, is smaller than ζ1 due to a negative charge of the marker 2 (for example, when ζ1=-10mV, ζ2=-11mV and ζ3=-20mV). As a result, particle motion in the electrokinetic phenomena such as electrophoresis and electroosmosis can be increased. In the case where a material being tested is a positively charged particle, it is possible to change the particle motion by using a marker having a positive charge.

The antibody specifically binding to the particle being tested can be prepared according to a method commonly known in the art, or a commercially available antibody can be utilized therefor. The above-stated antibody may be a polyclonal antibody, a monoclonal antibody, an antibody fragment, a single-stranded antibody, and a chimeric antibody. Such an antibody can specifically recognize an epitope of a biological functional substance selected from a group consisting of an organic polymer, a protein, sugar, lipid, and nucleic acid, which are present on a particle, and bind thereto. The antibody used in the present invention preferably shows the specific binding affinity of at least approximately 10⁵, 10⁶, 10⁷, 10⁸, 10⁹, or 10¹⁰M⁻¹ for the particle being tested if the affinity is represented by Ka (association constant)=[Ag-Ab]/([Ag]·[Ab])(where Ag denotes an antigen, Ab denotes an antibody, and Ag-Ab denotes an antigen-antibody complex).

Such an antibody can be prepared according to a method commonly known in the art: That is, a particle or a portion thereof is administered into a pad, a muscle, a skin, the surrounding of a lymph node, or an abdominal cavity of a host such as goat, sheep, cattle, guinea pig, rabbit, rat, and mouse, and immune globulin generated in the host is precipitated, isolated, and purified by a conventional method including affinity purification to obtain the antibody.

Alternatively, such an antibody can be obtained as a monoclonal antibody by using a technique commonly known in the art, such as the hybridoma technique first described by Koehler and Milstein (Nature 256:495, 1975), the human B-cell hybridoma technique (Kosbor et al., 1983, Immunol. Today 4 72; Cote et al., 1983, Proc. Natl. Acad. Sci. USA, 80:2026), and the EBV hybridoma technique (Cole et al., MONOCLONAL ANTIBODIES AND CANCER THERAPY, Alan, R Liss inc., New York, NY, pp.77-96, 1985). A specific procedure for obtaining a monoclonal antibody is described in Goding et al., MONOCLONAL ANTIBODIES: PRINCIPLES AND PRACTICE (second edition) Acad. Press, N. Y., for example.

A "functional equivalent" of the above-described antibody includes a fragment obtained by cutting only a binding site from an antibody specifically binding to the particle being tested. The antibody is composed of an H chain and an L chain, each having three antigen (corresponding to the target particle of the present invention) binding sites (CDR: complementary determinant region), and each binding site is capable of specifically binding to the antigen. The binding mode is similar to a protein (including a peptide)-protein binding mode (for example, a antigen-antibody binding or a peptide hormone-receptor binding) and is a multiple binding mode composed of a hydrogen bond, an ionic bond, and a hydrophobic bond. In general, it is possible to cut the above-described binding site, that is, to obtain a target peptide fragment, by generating a polynucleotide chain corresponding to each CDR from DNA or RNA coding for an antibody and expressing the polynucleotide chain by a molecular biological method commonly known in the art.

The antibody or its functional equivalent labeled by an appropriate marker (preferably, fluorescent substance) having a charge in a solution can be used as a charge control agent of the present invention. Typically, such a labeled antibody is bound to a biological functional substance such as an organic polymer, a protein, sugar, lipid, and nucleic acid, which are present on the cell surface, etc., by an ionic bond, a hydrophobic bond, or a hydrogen bond, or any combination thereof.

A non-antibody substance capable of binding to the particle being tested can be used as a charge control agent of the present invention. In the case where the target particle is a cell, for example, the above-stated non-antibody substance includes a ligand specifically binding to a receptor existing on the cell surface. Such a ligand includes, for example, a peptide hormone, a growth factor, cytokine, or catecholamine. Each of these molecules specifically binds to a receptor (cell surface receptor) present on a protoplasmic membrane, and develops its action. If the above-mentioned molecule itself has a charge in a solution, it can be used by itself as a charge control agent of the present invention. Also, a complex composed of a molecule having a function capable of binding to other substances and a marker having a charge in a solution can be used as a charge control agent of the present invention.

FIG. 1B schematically illustrates an exemplary case in which an aptamer is used as a charge control substance having a negative charge. A phosphate group of an aptamer is ionized in a solution, whereby the aptamer is negatively charged at physiological pH. As illustrated therein, an aptamer molecule is directly bound to the particle. Thus, a ζ potential ζ3 of the particle-aptamer complex is smaller than a ζ potential ζ1 of the particle itself. As a result, as is the case with FIG. 1A, particle motion in the electrokinetic phenomena such as electrophoresis and electroosmosis can be increased. In the case where a material being tested is a positively charged particle, it is possible to change the particle motion by using an aptamer having a positive charge.

As described above, the aptamer itself has a charge in a solution. Thus, even if the aptamer is not labeled by a marker having a charge, the aptamer itself can be used as a charge control agent of the present invention. Furthermore, it will be understood that a labeled aptamer, in which a marker having a charge is bound to an aptamer, can be used as a charge control agent of the present invention.

Note that a term "aptamer" used in the present specification has a meaning commonly used in the art, and indicates a substance binding to DNA in a cell for suppressing the activity of the target protein. Such a substance existing in a double-stranded DNA and a single-stranded RNA is known.

In general, the aptamer can be obtained by the following procedure.

First, oligo-DNAs (60 bases) are randomly synthesized. Next, an oligo-DNAbinding to the target protein is isolated by an affinity column from a pool of, in theory, more than ten trillion different oligo-DNAs. A process amplifying the isolated oligo-DNA by the polymese chain reaction (PCR), and re-isolating the amplified oligo-DNAs using the affinity column is repeatedly performed. By repeating this process five or more times, it is possible to select an aptamer having a close affinity for the target protein. Alternatively, if necessary, a commercially available aptamer can be utilized therefor. For example, an aptamer is available from Molecular Probe Inc. In general, the above-described aptamers are commercially available in a form of a solution or a powder.

In the case where an RNA aptamer or a DNA aptamer is used, a ribonuclease existing in a sample or a buffer breaks down the aptamer. As a result, a charge of the aptamer often changes. Thus, in general, a ribonuclease inhibitor (e.g., RNasin (R) (Promega Corp.)) is preferably added in advance.

On the other hand, in the case where a protein such as an antibody or a peptide is used, it is preferable that a protease inhibitor be appropriately added.

A marker for providing a charge (positive or negative) to a protein (e. g. , an antibody or a ligand for a cell surface receptor) or nucleic acid (e.g., an aptamer) which specifically binds to the target particle preferably includes a compound having a sulfonic group, a phosphate group, an amino group, an alcohol group, a phenol group, or a carboxylic acid group, for example, capable of ionizing in a solution, and having a function capable of binding to other substances.

Compounds having a sulfonic group and a function capable of binding to other substances include, for example, Cascade Blue (R), taurine, 2-hydroxy-5-sulfoanilinesalicylidene, 1-(2-hydroxy-4-sulfonaphtylazo) 2-naphtol-3, 6-disulfonicacid, Lucifer Yellow, Mordant Blue-31, and 5-sulfo-8-quinolinol, or derivatives of these compounds. Cascade Blue, 1-(2-hydroxy-4-sulfonaphtylazo) 2-naphtol-3, 6-disulfonic acid, Lucifer Yellow, Mordant Blue-31, or 5-sulfo-8-quinolinol, or a derivative of these compounds, in particular, is preferable since it is a fluorescent substance and can be utilized as a detector (dyeing marker).

Compounds having a phosphate group and a function capable of binding to other substances include, for example, adenosine monophosphate, uracil monophosphate, thymidylate monophosphate, guanidyl monophosphate, and Oregon Green 488, or derivatives of these compounds. Oregon Green 488 or a derivative thereof, in particular, is preferable since it is a fluorescent substance and can be utilized as a detector (dyeing marker).

As a compound having an amino group and a function capable of binding to other substances, a compound having a tertiary amino group or a quaternary amino group and having a function capable of binding to other substances is preferable.

Compounds having a tertiary amino group and a function capable of binding to other substances include, for example, dimethylethylenediamine, dimethylglycine, dimethylphenylene diammonium, calcein, dansyl chloride, and diethylaminocoumarin carboxylic acid hydrazide, or derivatives of these compounds. Calcein, dansyl chloride, or diethylaminocoumarin carboxylic acid hydrazide, or a derivative of these compounds, in particular, is preferable since it is a fluorescent substance and can be utilized as a detector (dyeing marker).

Compounds having a quaternary amino group and a function capable of binding to other substances include, for example, FM3-25 (R), betaine, cartinine, tetramethylrhodamine cadaverine, and rhodamineX, or derivatives of these compounds. FM3-25 (R), tetramethylrhodamine cadaverine, or rhodamineX, or a derivative of these compounds, in particular, is preferable since it is a fluorescent substance and can be utilized as a detector (dyeing marker).

Compounds having an alcohol group and a function capable of binding to other substances include, for example, aminoethanol, aminobutanol, aminopropanol, and aminobenzylalcohol.

Compounds having a phenol group and a function capable of binding to other substances include, for example, aminohydroxy benzoic acid, aminomethylphenol, 8-quinolinol, and salicylideneaminophenol, or derivatives of these compounds. 8-quinolinol or salicylideneaminophenol, or a derivative of these compounds, in particular, is preferable since it is a fluorescent substance and can be utilized as a detector (dyeing marker).

Compounds having a carboxylic acid group and a function capable of binding to other substances include, for example, aminobutyric acid, amino benzoic acid, and calcein. Calcein, in particular, is preferable since it is a fluorescent substance and can be utilized as a detector (dyeing marker).

A large number of dyeing markers are commercially available. In an embodiment which will be described below, a Cascade Blue (R) derivative and FM3-25 (R) are used.

Other markers binding to a molecule (e.g., an antibody, a ligand for a cell surface receptor, or an aptamer) specifically binding to the target particle and having a negative or positive charge include, for example, a gold colloid and latex.

A technique for labeling the above-described protein, peptide, and nucleic acid, etc., with these markers is commonly known in the art. In the case where a commercially available dyeing marker is used, the dyeing marker is generally bound to the above-described molecules as per the instructions prepared by a manufacturer.

When the charge control agent of the present invention is used, the optimum pH of a sample solution should be appropriately selected. For example, the pKa of a carboxylic acid group is approximately 2-5, whereby the carboxylic acid group is negatively charged since a proton is removed therefrom when the pH of the sample solution is in a range of 2-5 or higher. Thus, in order to cause the charge control agent having a carboxylic acid group to react with the target particle while keeping a negative charge, it is preferable that the pH of the sample solution be set so as to be 4 or higher.

Similarly, in the case where a charge control agent having a phenol group or an alcohol group is used, since the pKa of the phenol group and the alcohol group is approximately 9-11, it is preferable that the pH be set so as to be in a range of 9-11 or higher in order to use the agent while keeping a negative charge.

In the case where a charge control agent having a tertiary amine group is used, since the pKa of the tertiary amine group is approximately 10-12, it is preferable that the agent be used at the pH equal to or smaller than 10-12 in order to use it while keeping a negative charge.

On the other hand, a sulfonic group, a phosphate group, and a quaternary amino group have a charge in a solution of pH 2-13, whereby these groups can be used in a wide range of pH. When a negative charge is required, it is most preferable that a compound having a sulfonic or phosphate group be used. When a positive charge is required, it is most preferable that a compound having a quaternary amino group be used.

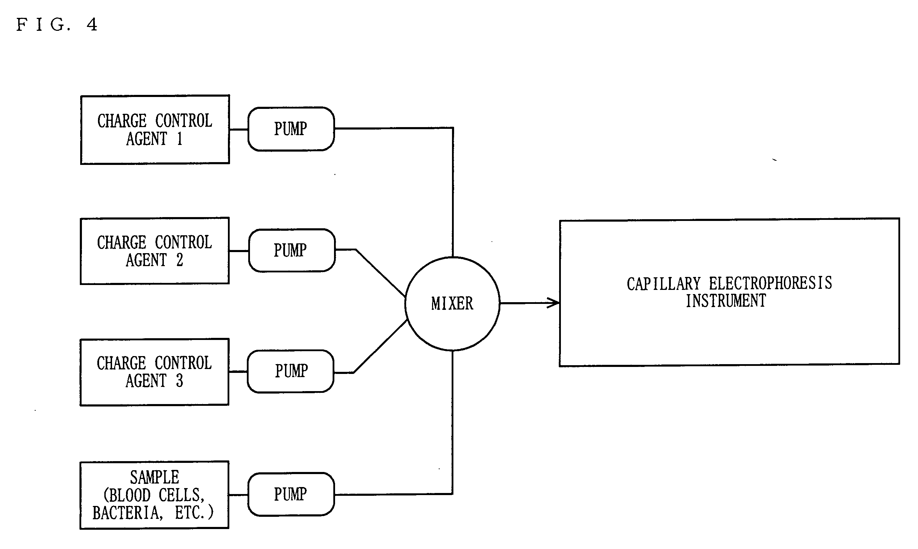

A method of separating and/or quantitatively determining the target particles whose surface charge is modified with the charge control agent of the present invention may be an arbitrary method by which the particles are separated or quantitatively determined based on the surface charge thereof. Typically, the capillary electrophoresis is most preferable. A commercially available capillary electrophoresis instrument can be used for achieving the object of the present invention. However, as schematically illustrated in FIG. 4 of the present application, the capillary electrophoresis instrument provided with a means for mixing a charge control agent and a sample is further preferable.

Other possible methods and devices for separating and/or quantitatively determining the target particles in a sample by using the charge control agent of the present invention include, for example, a method or device using a chip utilizing capillary electrophoresis.

In the case where a blood cell is an object being tested, the present invention can be used for the following applications.

### (Diagnosis of fatigue and stress)

It is commonly known that chronic fatigue, overwork, and severe mental stress affect the body' s immune system and increase the possibility of developing an infectious disease, etc. Thus, by testing cells of the immune system, it is possible to measure how working conditions currently affect a person's health, especially, to measure the level of accumulating fatigue or stress.

An analysis of a lymphocyte surface antigen, whose test is easy to perform, is known as a test for an immune function. It is known that acute stress increases the number and the activity of NK cells (natural killer cells), which are a type of lymphocyte, in the deletion blood and that chronic stress decreases the number and the activity thereof. For example, decreased activity of the NK cells has been reported among patients with depression (TOMONOBU Kawano (ed.) "Handbook of Stress Diagnosis", Medical Science International, January 1990, p11, table 2-2) . Also, it has been reported that stress associated with examinations decreases the activity of the NK cells and production of interferon (TOMONOBU Kawano (ed.) "Handbook of Stress Diagnosis", Medical Science International, January 1990, p11, table 2-2).

NK cells play a central role in a host defense system such as antitumor and antivirus immune response, for example. The activity of NK cells is present in advance, even if not exposed to a source of infection, and shows the properties unique to an innate defense system. Thus, NK cells act as a primary defense line in the defense system. By detecting the above-stated natural killer activity or a change in the number of NK cells, it is possible to detect a change in the immune function of a subject (for example, D. M. Weir, "Immunology", Kyoritu Publication, April 1999, pp. 36-37; TOMONOBU Kawano (ed.) "Handbook of Stress Diagnosis", Medical Science International, January 1990, p11, table 2-2).

CD antigens expressed on the NK cell surface include, for example, CD16, CD56, and CD57. CD56, in particular, can be used alone as a marker for NK cells since more than 90 percent thereof are found in NK cells. In order to measure the number of NK cells of a subject by using the charge control agent of the present invention, for example, a substance such as an antibody specifically binding to the above-described CD56 is modified by labeling it with a marker having a charge (that is, the charge control agent of the present invention is prepared), and the modified antibody is mixed with blood of the subject, thereby binding the modified antibody to the NK cells in the blood. Next, the NK cells to which the modified antibodies are bound are separated and quantitatively determined using the capillary electrophoresis, whereby it is possible to measure the number of NK cells.

### (Diagnosis of virus infection)

It is known that virus infection changes the composition of lymphocytes. For example, Rosenberg et al. have shown that HIV infection induces decline in the number of CD4-expressing T cells in blood and decline in CD4/CD8 ratios (Immunology Today, Vol. 19, Issue 1, 1998, pp.10-17). HIV infection decreases CD4-positive lymphocytes, and other virus infection increases CD8-positive lymphocytes. Thus, by detecting the increase and decrease of these lymphocytes, it is possible to detect whether or not a subject is infected with HIV or other viruses.

The above-described detection is performed by using the charge control agent of the present invention as follows: First, a substance (for example, an antibody) specifically binding to CD4 and CD8 is modified to provide a charge therewith (that is, the charge control agent of the present invention is generated), and a modified antibody is mixed with blood of the subject, thereby binding the modified antibody to the CD4- and CD8-positive lymphocytes in the blood. Next, the NK cells to which the modified antibodies are bound are separated and quantitatively determined using the capillary electrophoresis, whereby it is possible to measure the number of CD4- and CD8-positive lymphocytes.

On the other hand, in the case where a substance specifically binding to a virus being tested is used, it is possible to directly detect the number of viruses. For example, the charge control agent of the present invention obtained by binding a marker having a charge to an antibody specifically binding to the virus being tested is bound to the target virus, and separation and quantitative determination thereof is performed by the capillary electrophoresis, etc., whereby it is possible to directly measure the number of viruses.

On the other hand, in the case where an object being tested is a bacterium which is a microorganism, the present invention can be used for the following applications.

### (Prevention and diagnosis of food poisoning)

In the case where a bacterium which is a microorganism is an object being tested, the present invention can be applied to detection of food poisoning bacteria. The food poisoning bacteria include the following bacteria, each tends to contaminate a specific food.

Livestock, especially cattle, are often infected with Escherichia coliform bacillus, and beef contaminated with this bacteria generally causes food poisoning. Other than beef, all food including lettuce, radish sprouts, and water may cause this food poisoning.

Salmonella is by nature a zoonotic pathogen, and is commonly found in the intestine of livestock and fowl. Also, other than a chicken, a pig, and cattle, a pet such as a lizard and a tortoise may be a carrier. In the case where meat or eggs contaminated with salmonella is used as an ingredient, food poisoning may be caused by salmonella survived due to improper cooking and/or by those contaminating the cooked foods. Also, a kitchen or food may be contaminated with feces and urine of rodents infected with salmonella, thereby causing food poisoning. Food such as eel, fresh slices of raw liver, rolled egg, hand-made mayonnaise, and roasted chicken, especially livestock food product of meat and eggs, often cause food poisoning.

Yersinia enterocolitica is isolated from many animals such asmammals, birds, reptiles, and freshwater fish, and water. Thus, transmission of Yersinia enterocolitica to humans occurs by contact with an infected animal or by the ingestion of contaminated meat, especially pork.

Vibrio parahaemolyticus, which inhabits in seawater and sea mud, grows in large quantity in seawater at a water temperature of 20 degrees or higher and at a minimum temperature of 15 degrees or higher, and contaminates fish and seafood, thereby being carried on land therewith. Typically, food such as fresh slices of raw fish and seafood and sushi often cause food poisoning. However, vegetables pickled overnight may be contaminated with Vibrio parahaemolyticus through cooking equipment and fingers, etc., used for cooking raw fish and seafood.

Bacillus cereus is widely distributed in nature such as soil, dust, and water, and is commonly found in soil-related cereals, beans, and spices, etc. Food such as fried rice, spaghetti, fried Chinese noodle, and rice in omelet, etc., which are made with leftovers cooked or boiled the day before, may cause food poisoning.

Campylobacter is found in the intestine of livestock, fowl, and pets such as pigeons, etc., and is commonly found in chicken meat because chickens carry the above bacteria at high rates. The above bacterium is also found in pork and beef, and may be found in stream water and well water probably due to feces of the carrier animals such as wild birds and pets. Food poisoning is often caused by eating raw meat such as chicken fillet or eating inadequately cooked meat at the time of barbecue or beef barbecue. Also, salad and raw water, etc., may cause the food poisoning.

Clostridium perfringens, which is one type of Bacillus thuringiensis, is widely distributed in nature such as seawater, etc. , and is commonly found in the intestine of humans and animals. As called a "school lunch disease", curry, stew, and seasoning soy source for buckwheat noodle, which are cooked in large pots, may cause food poisoning.

Staphylococcus aureus is present in boils, athlete's foot, acnes, a throat or nose, a skin, and hair, etc., as well as in purulent wounds, and even a healthy person is a carrier thereof. Food is often contaminated there with through fingers, and therefore all foods may cause food poisoning. Boxed meals, Japanese cakes, cream puffs, and, in particular, rice balls often cause food poisoning.

In order to detect bacteria from the food contaminated therewith, for example, 225 ml of EC medium with novobiocin is added to 25 g of each food sample, and stomaching is performed therefor. A required amount is dispensed from the resultant solution (such a procedure is commonly used in the art), and is mixed with the charge control agent of the present invention generated using an antibody specifically binding to the bacteria being tested. After a bacteria-charge control agent complex is generated, the capillary electrophoresis is performed, whereby it is possible to isolate the above bacteria-charge control agent complex. As such, in the case where food poisoning occurs, it is possible to detect offending bacteria. Furthermore, by previously detecting such offending bacteria, it is possible to prevent food contaminated with these offending bacteria from being marketed.

Hereinafter, the present invention will be described using the following examples. These examples are illustrative and not restrictive.

### (Example 1)

### (Separation of blood cells)

A Cascade Blue (R) derivative (Molecular Probe Inc.: aminoethyl4-azidebenzamide trisodium salt: C₂₇H₁₈N₅Na₃O₁₂S₃: hereinafter simply referred to as a Cascade Blue derivative), as shown by a structural formula 1, is bound to an antibody for CD4 (cluster of differentiation 4) that is expressed exclusively on helper T cells, and an antibody for CD 8 that is expressed exclusively on killer T cells in accordance with the following procedure.

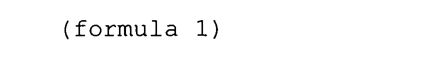

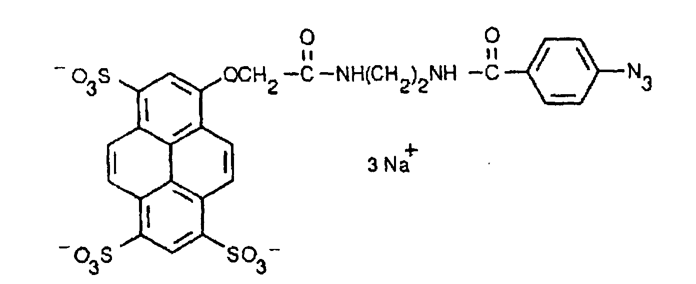

Note that, as shown in formula 1, the Cascade Blue derivative has a sulfonic group.

An anti-CD4 antibody (1 mg/ml, in phosphate buffer solution (PBS) at pH7.0) obtained from Sigma and a tenfold molar quantity of Cascade Blue derivative (5.1µ g/ml, in PBS at pH7.0) were mixed and left for 3 hours at room temperature, whereby the Cascade Blue derivative was bound to the anti-CD4 antibody. Next, the resultant reaction solution was subjected to gel filtration (SephadexG-25), thereby removing unbound Cascade Blue derivatives. As a result, an anti-CD4 antibody with two Cascade Blue derivatives bound thereto per molecule was obtained.

Similarly, an anti-CD8 antibody obtained from Sigma was processed, and an anti-CD8 antibody with eight Cascade Blue derivatives bound thereto per molecule was obtained.

Note that the number of Cascade Blue derivatives bound to the antibody was calculated by the following equation.

The number of Cascade Blue derivatives bound to the antibody=[Cb410 nm]/([Ab-Cb280 nm]-α×[410 nm])

Here,
[Ab280 nm]: absorbance of the antibody at 280 nm,
[Cb280 nm]: absorbance of the Cascade Blue derivative at 280 nm,
[Cb410 nm]: absorbance of the Cascade Blue derivative at 410 nm,
[Ab-Cb280 nm]: absorbance of the antibody labeled with Cascade Blue derivative at 280 nm, and
α=[Cb280 nm] / [Cb410 nm].

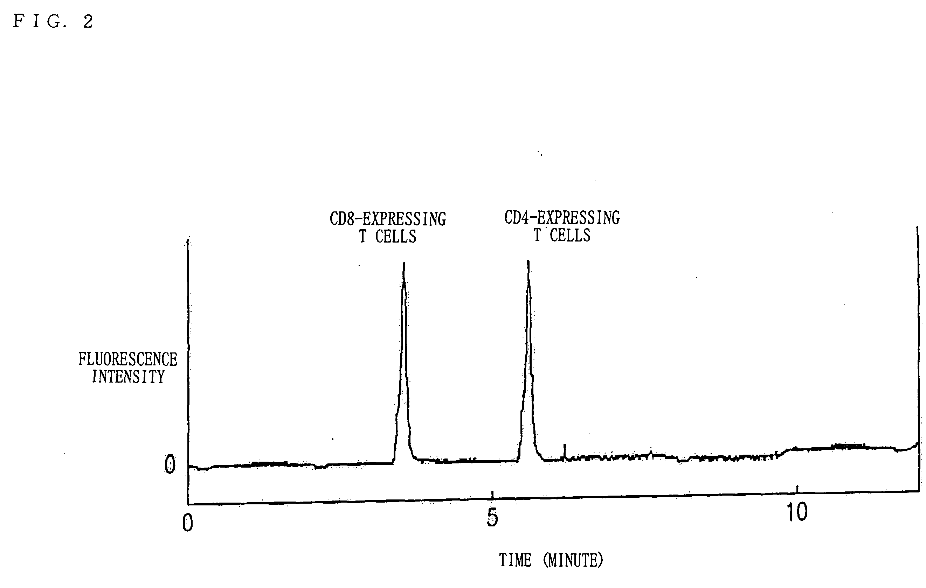

After these Cascade Blue derivative-labeled antibodies were incubated (at 25°C for 1 hour) with a sample containing T cells obtained from blood in accordance with a procedure described below in detail, the resultant mixture was injected into a capillary electrophoresis instrument (Beckman, P/ACE system MDQ). As a result, as shown in FIG. 2, separation of helper T cells and killer T cells was realized. Note that FIG. 2 shows the results of scanning of the intensity of Cascade Blue derivative-derived fluorescence at 430 nm by exciting the capillary after electrophoresis by light at 410 nm.

### (Contact of the charge control agent with blood cells and separation of blood cells)

500 µl of a solution containing CD4-expresisng cells and 500 µl of a solution containing CD8-expresisng cells were transferred to a 1.5 ml sample tube, and 5 µl of a Cascade Blue-labeled anti-CD4 antibody solution (1 mg/ml) and 5 µl of a Cascade Blue-labeled anti-CD8 antibody solution (1 mg/ml) were added thereto. After the resultant solution was left for 3 hours at room temperature in a dark place, the fluorescent capillary electrophoresis was performed therefor under the following conditions.

### <Capillary electrophoresis and detection conditions>

Instrument: P/ACE system MDQ (Beckman Coulter)
Capillary: fused silica capillary (Beckman) 100 µm inner diameter, 32.0 cm total length, and 21 cm effective length
Electrophoresis conditions: 10 kV, 20 minutes
Electrophoresis buffer solution: Tris base 10.8 g, boric acid 5.5 g, 0.5M EDTA (pH8) 4 ml, sodium alginate 0.1g, sodium chloride 2 g were dissolved in water, and the volume thereof was adjusted to 1000 ml
Detection conditions: excitation wavelength 410 nm, fluorescent wavelength 430 nm
Detector: MDQ laser-Induced Fluorescence (LIF) detector

As a result, respective peaks were detected at the same mobility as BacLightSYTO9 (Molecular Probe Inc.), and each peak fraction was collected and examined using an electron microscope, whereby respective types of cells generating fluorescence were identified. The results revealed that the Cascade Blue-labeled anti-CD4 antibody and the Cascade Blue-labeled anti-CD8 antibody specifically bound to the CD4 cells and CD8 cells, respectively, were detected. Note that, a peak of a control sample resulting from adding only the Cascade Blue-labeled anti-CD4 antibody solution and the Cascade Blue-labeled anti-CD8 antibody solution to 100mM Tris-boric acid buffer was detected at substantially the same residence time as the electroosmotic flow, and no other peak was detected.

### (Example 2)

### (Separation of bacteria)

As with Example 1, the Cascade Blue derivative was bound to an antibody (prepared according to a common procedure) for Salmonella typhimurium (hereinafter referred to as S. T.) and an antibody (prepared according to a common procedure) for Salmonella enteritidis (hereinafter referred to as S. E.). As a result, an anti-ST antibody with 1.5 Cascade Blue derivatives bound thereto per molecule and an anti-SE antibody with nine Cascade Blue derivatives bound thereto per molecule were obtained.

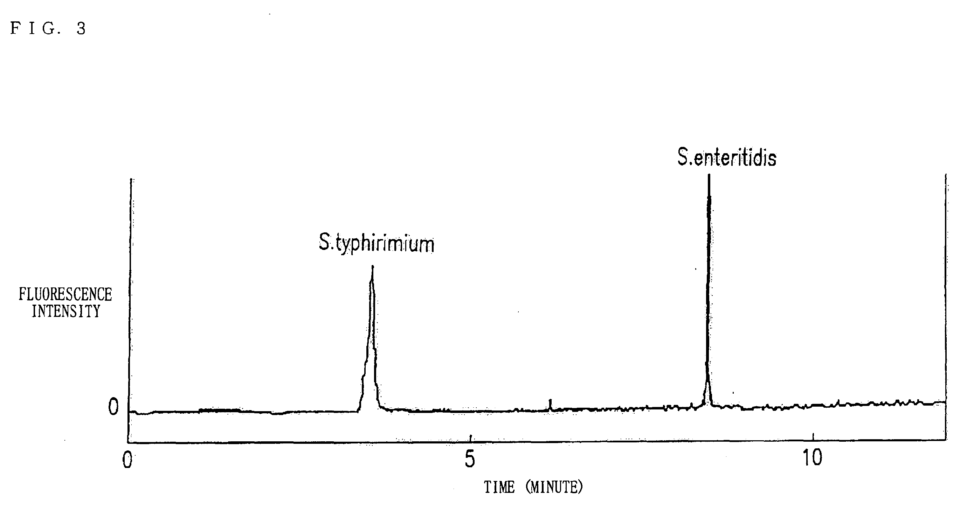

After these Cascade Blue derivative-labeled antibodies were incubated (at 37°C for 30 minutes) with a sample containing the salmonellae in accordance with a procedure described below in detail, the resultant mixture was injected into the capillary electrophoresis instrument (Beckman, P/ACE system MDQ). As a result, as shown in FIG. 3, separation of each salmonella was realized. Note that FIG. 3 shows the results of scanning of the intensity of fluorescence of a wavelength of 430 nm derived from Cascade Blue derivative while exciting the capillary after electrophoresis by light of a wavelength of 410 nm.

### (Contact of the charge control agent with salmonella and separation of salmonella)

A platinum loopful of bacterial cell body removed from a slant culture of S. T. was separately inoculated into a 200 ml conical flask containing 50 ml of Enterbacteriaceae Enrichment Mannitol broth (EEM broth: 4.35 g/100 ml) prepared according to a common procedure, and was incubated with shaking at 37°C for 16 hours. Similarly, a culture solution of S. E. was prepared. Each 500 µl of the resultant culture solution was transferred to a 1. 5 ml sample tube, and 5 µl of a Cascade Blue-labeled anti-ST antibody solution and 5 µl of a Cascade Blue-labeled anti-SE antibody solution were added thereto and left for 30 minutes at 37°C in a dark place, thereby bringing salmonella into contact with a labeled antibody for each salmonella. Next, the fluorescent capillary electrophoresis was performed therefor under the following conditions.

### <Capillary electrophoresis and detection conditions>

Instrument: P/ACE system MDQ (Beckman Coulter)
Capillary: fused silica capillary (Beckman) 75 µm inner diameter, 31.2 cm total length, and 21 cm effective length
Electrophoresis conditions: 10 kV, 20 minutes
Electrophoresis buffer solution: Tris base 10.8 g, boric acid
5. 5 g, 0.5M EDTA (pH8) 4 ml, sodium alginate 0.1 g, sodium chloride 2 g were dissolved in water, and the volume thereof was adjusted to 1000 ml
Detection conditions: excitation wavelength 410 nm, fluorescent wavelength 430 nm
Detector: MDQ laser-Induced Fluorescence (LIF) detector

Also, the fluorescent capillary electrophoresis was similarly performed for a solution (control sample) generated by adding 5 µl of a solution containing either of the above two Cascade Blue-labeled anti-salmonella antibodies to 500 µl of 100mM Tris-boric acid buffer, and for a solution (comparison sample) generated as follows: each of ten types of bacteria including Brucella sp. strain KYM-1, Stenotrophomonas sp. strain KYM2, Acinetobactersp. strain KYM3, Commanonas sp. strain KYM4, Aureobacterium sp. strain KYM6, Cellulomonas sp. strain KYM7, Acinetobacterium sp. strain KYM8, and three types of Escherichia coli was dissolved in 500 µl of 100mM Tris-boric acid buffer so as to be 10⁵/ml, 5 µl of a solution containing either of the above two Cascade Blue-labeled anti-salmonella antibodies was added thereto, and the resultant solution was left for 15 minutes at room temperature in a dark place. As a result, as for the control sample containing only the Cascade Blue-labeled anti-salmonella antibody in the Tris-boric acid buffer, a peak of the free Cascade Blue-labeled anti-salmonella antibody was detected at substantially the same residence time as the electroosmotic flow, and no other peak was detected. As is the case with the control, as for the solution (comparison sample) containing ten types of bacteria, only a peak of the free Cascade Blue-labeled anti-salmonella antibody was detected.

On the other hand, as for a sample containing salmonella, other than a peak corresponding to the free Cascade Blue-labeled anti-salmonella antibody, other peaks were detected at the same mobility as BacLightSYTO9, and the above peak fractions were collected and examined using an electron microscope, whereby each salmonella generating fluorescent was identified. The results revealed that each of the Cascade Blue-labeled anti-salmonella antibodies specifically bound to corresponding salmonella was detected.

### (Example 3)

As with Example 1, separation of helper T cells and killer T cells was performed, except that FM (R) 3-25: N-(3-triethlyammoniumpropyl)-4-(4-(dioctadecylamino) styryl) pyridiniumdi-4-chlorobenzenesulfonate (C₆₈H₁₁₃Cl₂N₃O₆S₂) having a positive charge, as shown by a structural formula 2, was used in place of the Cascade Blue derivative. The capillary after electrophoresis was excited by light of a wavelength of 510 nm, and the intensity of fluorescence of a wavelength of 624 nm derived from the above derivative was scanned, whereby separation and detection of the helper T cells and killer T cells was realized.

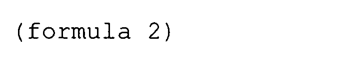

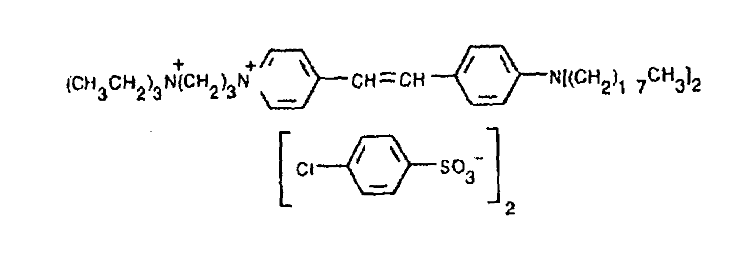

### (Example 4)

As with Example 1, separation of blood cells was performed using a particle separator whose structure is briefly illustrated in FIG. 4. Here, a CD4 antibody to which two Cascade Blue derivatives are bound was stored in a chamber indicated by a charge control agent 1 in FIG. 4, a CD8 antibody to which ten Cascade Blue derivatives are bound was stored in a chamber indicated by a charge control agent 2 in FIG. 4, a CD45 antibody to which twenty Cascade Blue derivatives are bound was stored in a chamber indicated by a charge control agent 3 in FIG. 4, and a blood sample was stored in a chamber indicated by a sample in FIG. 4. All pumps illustrated in the drawing were activated for mixing the labeled antibodies by a mixer connected to the pumps, and then the resultant solution was injected into the capillary electrophoresis instrument. The capillary after electrophoresis was excited by light of a wavelength of 410 nm, and the intensity of fluorescence of a wavelength of 430 nm derived from the above compound was scanned, whereby separation and detection of the helper T cells and killer T cells was realized.

### (Example 5)

### (Separation of Gram-positive bacteria)

The Cascade Blue derivative was bound to an antibody (prepared according to a common procedure) for Streptococcus Thermophilus (hereinafter referred to as ST) and an antibody (prepared according to a common procedure) for Streptococcus Mutans (hereinafter referred to as SM). As a result, an anti-ST antibody with two Cascade Blue derivatives bound thereto per molecule and an anti-SM antibody with five Cascade Blue derivatives bound thereto per molecule were obtained.

After these Cascade Blue derivative-labeled antibodies were incubated (at 37°C for 30 minutes) with a sample containing the above respective streptococci in accordance with a procedure described below in detail, the resultant mixture was injected into the capillary electrophoresis instrument (Beckman, P/ACE system MDQ). As a result, separation of each streptococcus was realized (not shown). Detection was performed as follows: the excitation was performed by light of a wavelength of 410 nm, and the intensity of fluorescence of a wavelength of 430 nm derived from Cascade Blue derivative was measured.

### (Contact of the charge control agent with streptococcus and separation of the bacteria)

A platinum loopful of bacterial cell body was removed from a slant solid culture of ST, separately inoculated into a 200 ml conical flask tryptic soybean culture prepared according to a common procedure, and incubated in static culture at 37°C for 16 hours in atmosphere of 95% CO2. Similarly, a culture solution of SM was prepared. Each 500 µl of the resultant culture solution was transferred to a 1.5 ml sample tube, and 5 µl of Cascade Blue-labeled anti-ST antibody solution (1 mg/ml) and 5 µl of Cascade Blue-labeled anti-SM antibody solution (1 mg/ml) were added thereto and left for 30 minutes at 37°C in a dark place, thereby bringing streptococci into contact with a labeled antibody for each streptococcus. Next, the fluorescent capillary electrophoresis was performed therefor under the following conditions.

### <Capillary electrophoresis and detection conditions>

Instrument: P/ACE system MDQ (Beckman Coulter)
Capillary: fused silica capillary (Beckman) 75 µ m inner diameter, 31.2 cm total length, and 21 cm effective length
Electrophoresis conditions: 10 kV, 20 minutes
Electrophoresis buffer solution: Tris base 10.8 g, boric acid 5.5 g, 0.5M EDTA (pH8) 4 ml, sodium alginate 0.1g, sodium chloride 2 g were dissolved in water, and the volume thereof was adjusted to 1000 ml
Detection conditions: excitation wavelength 410 nm, fluorescent wavelength 430 nm
Detector: MDQ laser-Induced Fluorescence (LIF) detector

Also, the fluorescent capillary electrophoresis was similarly performed for a solution (control sample) generated by adding 5 µl of a solution containing either of the above two Cascade Blue-labeled anti-streptococcus antibodies to 500 µl of 100mM Tris-boric acid buffer, and for a solution (comparison sample) generated as follows: each of ten types of bacteria including Brucella sp. strain KYM-1, Stenotrophomonas sp. strain KYM2, Acinetobactersp. strain KYM3, Commanonas sp. strain KYM4, Aureobacterium sp. strain KYM6, Cellulomonas sp. strain KYM7, Acinetobacterium sp. strain KYM8, and three types of Escherichia coli was dissolved in 500 µl of 100mM Tris-boric acid buffer so as to be 10⁷/ml, 5 µl of a solution containing either of the above two Cascade Blue-labeled anti-streptococcus antibodies was added thereto, and the resultant solution was left for 15 minutes at room temperature in a dark place. As a result, as for the control sample containing only the Cascade Blue-labeled anti-salmonella antibody in the Tris-boric acid buffer, a peak of the free Cascade Blue-labeled anti-salmonella antibody was detected at substantially the same residence time as the electroosmotic flow, and no other peak was detected. As is the case with the control, as for the solution (comparison sample) containing ten types of bacteria, only a peak of the free Cascade Blue-labeled anti-salmonella antibody was detected.

On the other hand, as for a sample containing streptococcus, other than a peak corresponding to the free Cascade Blue-labeled anti-streptococcus antibody, other peaks were detected at the same mobility as BacLightSYTO9, and the above peak fractions were collected and examined using an electron microscope, whereby each streptococcus generating fluorescent was identified. The results revealed that each of the Cascade Blue-labeled anti-streptococcus antibodies was specif ically bound to corresponding streptococcus, and then detected.

### INDUSTRIAL APPLICABILITY

The charge control agent of the present invention is useful to modify the amount of charges on the surface of target particles in a sample. The present invention is also useful to separate or quantitatively determine the target particles in the sample. The present invention is useful as a simple and accurate particle separation technology.

Also, the present invention is useful to check for stress and virus infection, and is useful for prevention and investigation, for example, of food poisoning.

## Claims

1. A composition for modifying an amount of charges on a surface of a target particle in a sample and separating or quantitatively determining the target particle in the sample, based on the modified surface charge amount, the composition comprising a charge control agent having a positive or negative charge in a solution and being capable of specifically binding to the target particle.

2. The composition according to claim 1, wherein the charge control agent specifically binds to a biological functional substance selected from a group consisting of an organic polymer, a protein, sugar, lipid, and nucleic acid, which are present on the surface of the target particle.

3. The composition according to claim 1, wherein the charge control agent comprises a group selected from a group consisting of a carboxylic acid group, a phosphate group, a sulfonic group, a phenol group, an alcohol group, a tertiary amino group, and a quaternary amino group.

4. The composition according to claim 2, wherein the charge control agent comprises a protein, a peptide, or nucleic acid, which is capable of specifically binding to the target particle.

5. The composition according to claim 4, wherein the nucleic acid is an aptamer or a functional equivalent thereof.

6. The composition according to claim 4, wherein the charge control agent further comprises a marker having a positive or negative charge in a solution.

7. The composition according to claim 6, wherein the charge control agent is a complex composed of an antibody or a functional equivalent thereof, which is capable of specifically binding to the biological functional substance, and the marker bound thereto.

8. The composition according to claim 6, wherein the charge control agent is a complex composed of a ligand for a receptor present on the surface of the target particle or a functional equivalent thereof and the marker bound thereto.

9. The composition according to claim 8, wherein the ligand is a peptide hormone, a growth factor, cytokine, or catecholamine.

10. The composition according to claim 6, wherein the charge control agent is a complex composed of an aptamer or a functional equivalent thereof and the marker bound thereto.

11. The composition according to claim 6, wherein the marker is a dyeing marker, a gold colloid, or latex.

12. The composition according to claim 11, wherein the dyeing marker is aminoethyl4-azidebenzamide trisodium salt or N-(3-triethlyammoniumpropyl)-4-(4-(dioctadecylamino) styryl) pyridiniumdi-4-chlorobenzenesulfonate.

13. The composition according to claim 1, wherein the charge control agent is reversibly bound to the target particle.

14. The composition according to claim 1, wherein the charge control agent is specifically bound to the target particle by an ionic bond or a hydrogen bond.

15. The composition according to claim 1, wherein the target particle is a cell selected from a group consisting of a white blood cell, a lymphocyte, a platelet, and a red blood cell.

16. The composition according to claim 15, wherein the lymphocyte is a T cell, a B cell, or an NK cell.

17. The composition according to claim 16, which is used for testing an immune function of a subject.

18. The composition according to claim 16, which is used for measuring a level of fatigue or stress of a subject.

19. The composition according to claim 16, which is used for determining whether or not a subject is infected with a virus.

20. The composition according to claim 1, wherein the target particle is a bacterium, a virus, or a fungus.

21. The composition according to claim 20, wherein the bacterium is selected from a group consisting of Escherichia coliform bacillus, salmonella, Yersinia enterocolitica, Vibrio parahaemolyticus, bacillus cereus, Campylobacter, Clostridium perfringens, and Staphylococcus aureus.

22. The composition according to claim 21, which is used for prevention and investigation of food poisoning.

23. A manufacturing method of a charge control agent used for modifying an amount of charges on a surface of a target particle in a sample and separating or quantitatively determining the target particle in the sample, based on the modified surface charge amount, the method comprising the step of:
binding a marker having a positive or negative charge in a solution to a protein, a peptide, or nucleic acid, or a functional equivalent thereof, which is capable of specifically binding to the target particle.

24. The method according to claim 23, wherein the protein, the peptide, or the nucleic acid, or the functional equivalent thereof is specifically bound to a biological functional substance selected from a group consisting of an organic polymer, a protein, sugar, lipid, and nucleic acid, which are present on the surface of the target particle.

25. The method according to claim 24, wherein the protein is an antibody.

26. The method according to claim 24, wherein the nucleic acid is an aptamer.

27. The method according to claim 24, wherein the protein or the peptide is a ligand for a receptor present on the surface of the target particle.

28. The method according to claim 23, wherein the marker comprises a group selected from a group consisting of a carboxylic acid group, a phosphate group, a sulfonic group, a phenol group, an alcohol group, a tertiary amino group, and a quaternary amino group.

29. The method according to claim 23, wherein the marker is a dyeing marker, a gold colloid, or latex.

30. The method according to claim 29, wherein the dyeing marker is aminoethyl4-azidebenzamide trisodium salt or N-(3-triethlyammoniumpropyl)-4-(4-(dioctadecylamino) styryl) pyridiniumdi-4-chlorobenzenesulfonate.

31. The method according to claim 23, wherein the target particle is a cell selected from a group consisting of a white blood cell, a lymphocyte, a platelet, and a red blood cell.

32. The method according to claim 23, wherein the target particle is a bacterium, a virus, or a fungus.

33. The method according to claim 23, wherein a ratio or an amount of the marker to be bound to the protein, the peptide, or the nucleic acid, or the functional equivalent thereof is adjustable.

34. A method of separating or quantitatively determining a target particle in a sample, comprising the steps of:
mixing a sample containing the target particle and a charge control agent specifically binding to the target particle and having a positive or negative charge in the sample, and binding the charge control agent to the target particle; and
separating or quantitatively determining the target particle provided with the charge control agent bound thereto, based on a surface charge modified by the binding of the charge control agent, by applying a voltage or current to the sample resulting from the mixing.

35. The method according to claim 34, wherein the mixing step is separately performed for a plurality of types of particles.

36. The method according to claim 34, wherein the mixing step is performed for mixing a plurality of types of particles with respective charge control agents which are different from each other.

37. The method according to claim 34, wherein the target particle is a cell selected from a group consisting of a white blood cell, a lymphocyte, a platelet, and a red blood cell, or a microorganism selected from a group consisting of a bacterium, a virus, and a fungus.

38. The method according to claim 37, wherein the charge control agent is bound to a biological functional substance selected from a group consisting of an organic polymer, a protein, sugar, lipid, and nucleic acid, which are present on the surface of the target particle.

39. The method according to claim 38, wherein the charge control agent comprises a protein, a peptide, or nucleic acid, which is capable of specifically binding to the target particle.

40. The method according to claim 39, wherein the nucleic acid is an aptamer or a functional equivalent thereof.

41. The method according to claim 39, wherein the charge control agent further comprises a marker having a positive or negative charge in a solution.

42. The method according to claim 41, wherein the charge control agent is a complex composed of an antibody or a functional equivalent thereof, which is capable of specifically binding to the biological functional substance, and the marker bound thereto.

43. The method according to claim 41, wherein the charge control agent is a complex composed of a ligand for a receptor present on the surface of the target particle or a functional equivalent thereof and the marker bound thereto.

44. The method according to claim 43, wherein the ligand is a peptide hormone, a growth factor, cytokine, or catecholamine.

45. The method according to claim 41, wherein the charge control agent is a complex composed of an aptamer or a functional equivalent thereof and the marker bound thereto.

46. The method according to claim 41, wherein the marker is a dyeing marker, a gold colloid, or latex.

47. The method according to claim 46, wherein the dyeing marker is aminoethyl4-azidebenzamide trisodium salt or N-(3-triethlyammoniumpropyl)-4-(4-(dioctadecylamino) styryl) pyridiniumdi-4-chlorobenzenesulfonate.

48. An instrument for separating or quantitatively determining a target particle in a sample, comprising:
mixing means for mixing a sample containing the target particle and a charge control agent specifically binding to the target particle and having a positive or negative charge in the sample, and binding the charge control agent to the target particle; and
separation/quantitative determination means for separating or quantitatively determining the target particle provided with the charge control agent bound thereto, based on a surface charge modified by the binding of the charge control agent, by applying a voltage or current to the sample resulting from the mixing.

49. The instrument according to claim 48, further comprising a plurality of injection means for separately injecting the sample containing the target particle and the charge control agent.

50. The instrument according to claim 48, wherein the target particle is a cell selected from a group consisting of a white blood cell, a lymphocyte, a platelet, and a red blood cell, or a microorganism selected from a group consisting of a bacterium, a virus, and a fungus.

51. The instrument according to claim 50, wherein the charge control agent is bound to a biological functional substance selected from a group consisting of an organic polymer, a protein, sugar, lipid, and nucleic acid, which are present on the surface of the target particle.

52. The instrument according to claim 51, wherein the charge control agent comprises a protein, a peptide, or nucleic acid, which is capable of specifically binding to the target particle.

53. The instrument according to claim 52, wherein the nucleic acid is an aptamer or a functional equivalent thereof.

54. The instrument according to claim 52, wherein the charge control agent further comprises a marker having a positive or negative charge in a solution.

55. The instrument according to claim 54, wherein the charge control agent is a complex composed of an antibody or a functional equivalent thereof, which is capable of specifically binding to the biological functional substance, and the marker bound thereto.

56. The instrument according to claim 54, wherein the charge control agent is a complex composed of a ligand for a receptor present on the surface of the target particle or a functional equivalent thereof and the marker bound thereto.

57. The instrument according to claim 56, wherein the ligand is a peptide hormone, a growth factor, cytokine, or catecholamine.

58. The instrument according to claim 54, wherein the charge control agent is a complex composed of an aptamer or a functional equivalent thereof and the marker bound thereto.

59. The instrument according to claim 54, wherein the marker is a dyeing marker, a gold colloid, or latex.

60. The instrument according to claim 59, wherein the dyeing marker is aminoethyl4-azidebenzamide trisodium salt or N-(3-triethlyammoniumpropyl)-4-(4-(dioctadecylamino) styryl) pyridiniumdi-4-chlorobenzenesulfonate.
